# EUROPEAN PATENT SPECIFICATION

(11) **EP 4 718 000 B1**
(45) Date of publication and mention of the grant of the patent: **18.11.2026**
(21) Application number: 25737984.2
(22) Date of filing: 03.01.2025
(51) Int. Cl.: A01N 1/145, C12N 5/00, F25D 11/04, F25D 13/06, F25D 25/04, G07F 9/10, G07F 11/54

(54) **FLOOR-STANDING CRYOGENIC STORAGE REFRIGERATOR**
BODENKÜHLSCHRANK ZUR KRYOGENEN LAGERUNG
RÉFRIGÉRATEUR DE STOCKAGE CRYOGÉNIQUE POSÉ AU SOL

(30) Priority: 29.07.2024 CN 202411021604
(43) Date of publication of application: 01.04.2026
(73) Proprietor: SHANGHAI ORIGINCELL BIOLOGICAL CRYO EQUIPMENT CO., LTD., Shanghai 201203 (CN)
(72) Inventor: QU, Jianguo, Shanghai 201203 (CN); XUE, Yunfeng, Shanghai 201203 (CN)
(74) Representative: Bayramoglu et al.
(86) International application number: PCT/CN2025/070480
(87) International publication number: WO 2026/025812

(56) References cited:
- CN-B- 109 399 043
- US-A1- 2010 028 214
- US-B2- 9 709 314

## Description

### TECHNICAL FIELD

The present invention relates to the technical field of sample storage, and in particular to a vertical low-temperature storage refrigerator.

### BACKGROUND

In the field of biological sample storage, cryogenic storage and retrieval devices are used for low-temperature storage of biological samples such as blood samples, vaccines, bacterial and viral strains, which enables to maintain viability of the samples at low temperatures during storage.

A cryogenic refrigerator disclosed in CN 113819699 A employs a hinged door. For the purpose of sample storage and retrieval, the entire hinged door must be opened normally, resulting in exposure of a cryogenic zone behind the door. Frequent storage and retrieval to objects stored in the cryogenic zone cause temperature variations and adversely affect the objects stored therein. Particularly in the field of biological sample storage, biological samples are highly sensitive to temperature variations, and easily lose viability. Therefore, a vertical low-temperature storage refrigerator of the present invention is designed.

US 2010/0028214 A1 discloses a tube picking mechanism, which stores biological or chemical samples. The samples are contained in storage tubes held in SBS footprint storage racks that are loaded into trays located within an ultra-low temperature freezer compartment. A tube picking mechanism resides in a tube picking chamber that is located adjacent the freezer compartment. The tube picking mechanism includes a cache within the tube picking chamber to facilitate fast paced shuttling of the tube racks from the freezer compartment into the tube picking chamber. The shuttle has a clamping mechanism to secure a tube rack in place when a gripper head picks a tube from the rack. The system also includes a push pin that pushes on the bottom of the respective tube as it is being picked from the tube rack. A one-dimensional bar code reader is included within the tube picking chamber. The gripper head is able to move vertically and rotate within the field of view of the one-dimensional bar code reader in order to facilitate identification and reading of one-dimensional bar codes located on the sidewall of picked storage tubes.

### SUMMARY

An objective of this section is to summarize some aspects of examples of the present invention and briefly introduce some preferred examples. Some simplifications or omissions may be made in this section, while such simplifications or omissions shall not be construed as limiting the scope of the present invention as defined in the appended claims.

In view of the above problems or problems in the prior art, the present invention is provided.

Therefore, an objective of the present invention is to provide a vertical low-temperature storage refrigerator, and the refrigerator enables sample scooping without the need to open an entire chamber door. Sample storage and retrieval are achieved only by driving a transfer window through a roller shutter to a corresponding basket access port, thereby avoiding exposure of other samples on the basket and improving the viability of the samples stored.

In order to solve the above technical problems, the present invention adopts the following technical solution: a vertical low-temperature storage refrigerator as presented in the appended claims.

The present invention has the following beneficial effects: in the present invention, the rolling storage member drives the transfer window to move upward or downward, so as to match the corresponding storage slot on the basket in the sample storage chamber, and the sample scooping mechanism penetrates the transfer window by moving upward or downward to scoop the sample on the basket, which avoids exposure of other samples that do not need to be extracted, and reduces the adverse effects on the samples in the sample storage chamber; the grasping mechanism, the transfer tank retention zone, and the code scanning mechanism are arranged together, such that the grasping mechanism facilitates sample grasping, transport and scanning, and only one grasping mechanism is needed to realize the free movement of a plurality of functional areas; and the thermal insulation door is configured to close the roller shutter passage assembly, so as to further insulate the sample storage chamber.

### BRIEF DESCRIPTION OF THE DRAWINGS

To describe the technical solutions in the examples of the present invention more clearly, the accompanying drawings required for describing the examples are briefly described below. Apparently, the accompanying drawings in the following description show some examples of the present invention, and those of ordinary skill in the art may still derive other drawings from these accompanying drawings without creative efforts. The accompanying drawings are detailed below:
FIG. 1 is a schematic diagram of an overall structure of a vertical low-temperature storage refrigerator of the present invention.
FIG. 2 is a front view of a vertical low-temperature storage refrigerator.
FIG. 3 is a sectional view of the vertical low-temperature storage refrigerator in FIG. 2 in an A-A direction.
FIG. 4 is a schematic diagram of a sample scooping mechanism of a vertical low-temperature storage refrigerator.

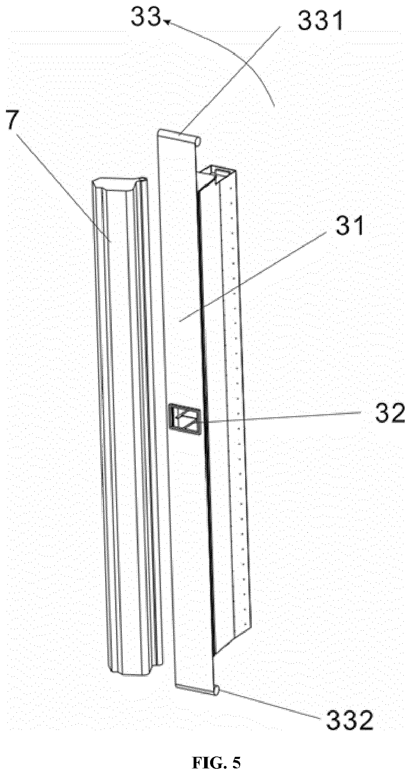

FIG. 5 is a schematic diagram of a roller shutter passage assembly of a vertical low-temperature storage refrigerator.

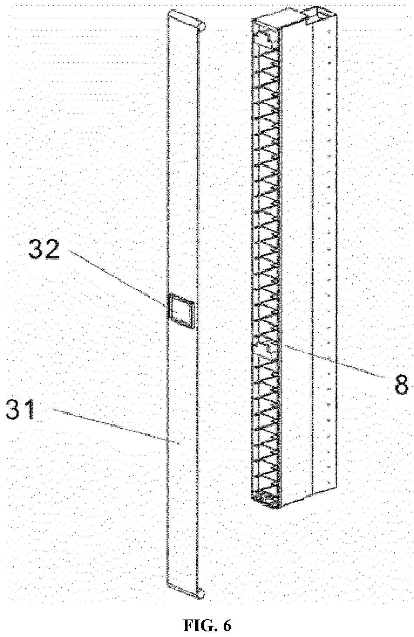

FIG. 6 is a schematic diagram of a basket of a vertical low-temperature storage refrigerator.

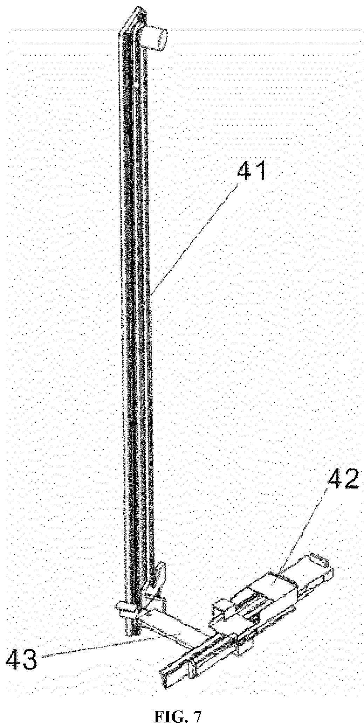

FIG. 7 is an enlarged schematic diagram of a sample scooping mechanism of a vertical low-temperature storage refrigerator.

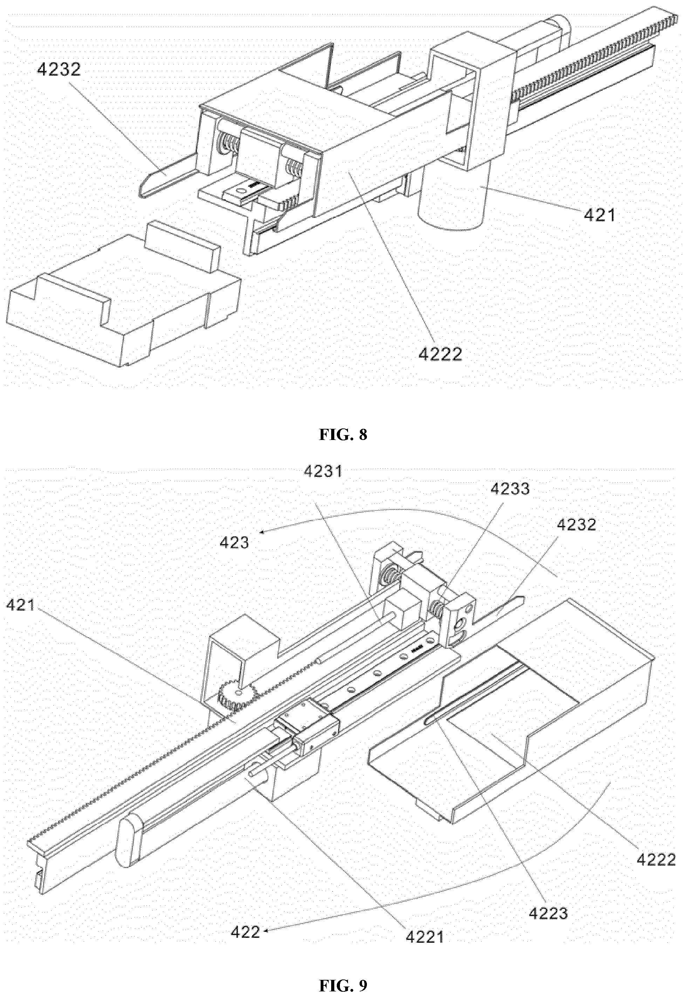

FIG. 8 is an enlarged schematic diagram of a pick-and-place gripper assembly of a vertical low-temperature storage refrigerator.
FIG. 9 is an exploded view of a pick-and-place gripper assembly of a vertical low-temperature storage refrigerator.

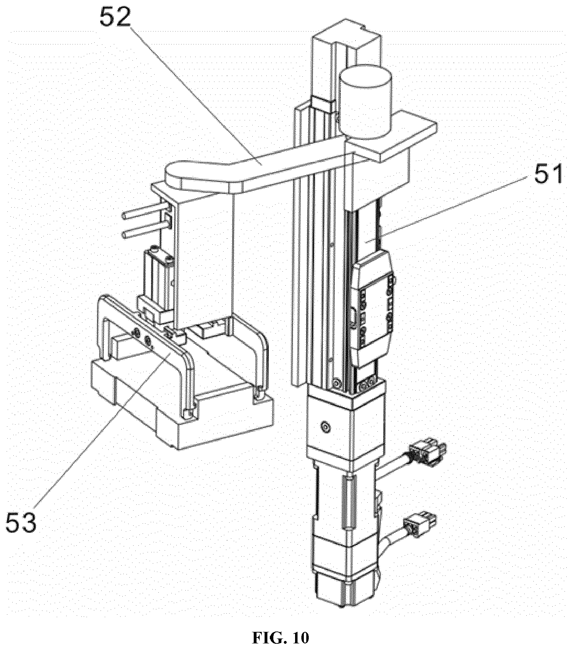

FIG. 10 is a schematic diagram of a grasping mechanism of a vertical low-temperature storage refrigerator.

Reference numerals in the figures: 1. sample storage chamber; 2. operation chamber; 3. roller shutter passage assembly; 4. sample scooping mechanism; 31. roller shutter; 32. transfer window; 33. rolling storage member; 331. upper roller shaft; 332. lower roller shaft; 5. grasping mechanism; 51. grasping lifting assembly; 52. grasping rotating assembly; 53. gripper member; 991. transfer tank; 992. tank operation door; 6. code scanning mechanism; 41. sample pick-and-place lifting assembly; 42. pick-and-place gripper assembly; 43. steering support member; 421. primary telescopic member; 422. secondary limiting and clamping member; 423. grasping member; 4221. secondary driving member; 4222. support frame; 4223. limiting bar; 4231. pushing member; 4232. scoop plate gripper; 4233. tension-adjusting member; 7. thermal insulation door; 8. basket; 993. observation window; and 994. operation terminal.

### DETAILED DESCRIPTION OF EMBODIMENTS

In order to enable the above objectives, features, and advantages of the present invention to be more apparent and easily understood, specific embodiments of the present invention will be described in detail below with reference to the accompanying drawings of the specification.

Numerous specific details are set forth in the following description to facilitate a thorough understanding of the present invention. However, the present invention can be practiced in many other ways than those described herein, and those skilled in the art may make similar improvements without departing from the scope of the invention as defined in the claims, and therefore the present invention is not limited by the specific examples disclosed below.

Furthermore, the term "an example" or "example" described herein refers to a specific feature, structure, or characteristic that may be included in at least one embodiment of the present invention. The term "in an example" that appears in different places in this specification neither necessarily refers to the same example, nor refers to a separate or selective example that is mutually exclusive with other examples.

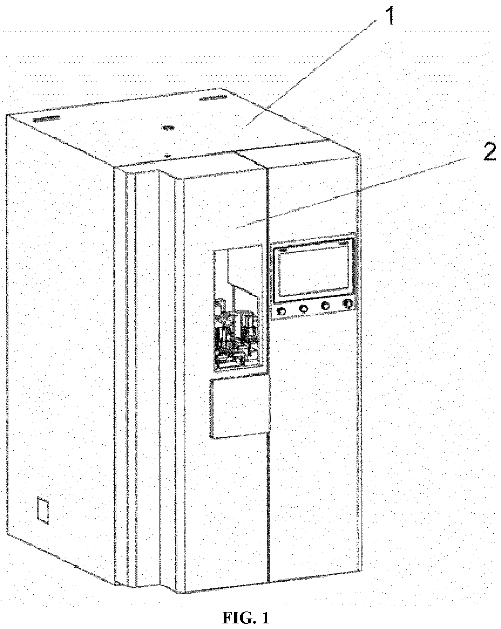

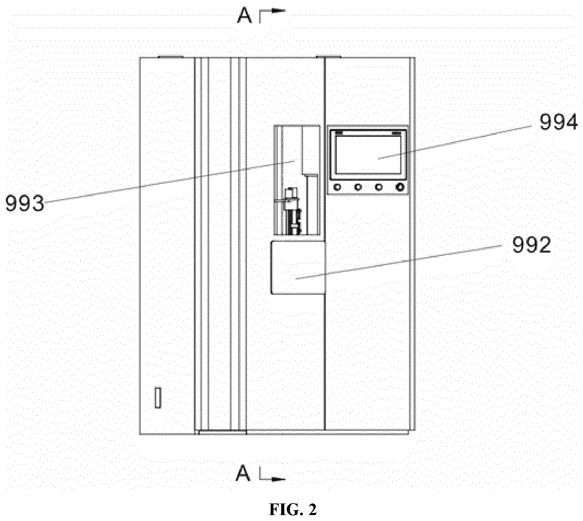

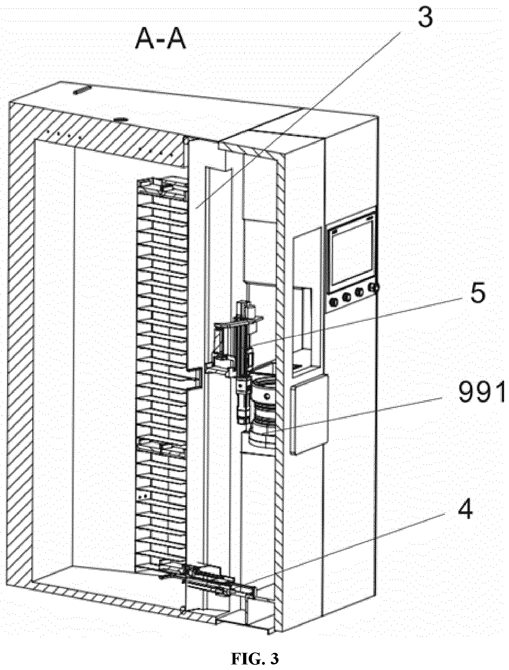

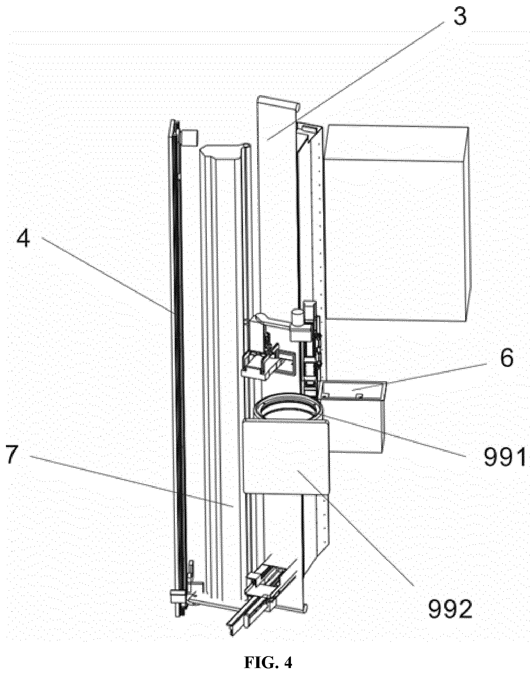

With reference to FIGs. 1-4, in a first example of the present invention, a vertical low-temperature storage refrigerator is provided, including a sample storage chamber 1 and an operation chamber 2, where the sample storage chamber 1 is configured for low-temperature storage of samples, and the operation chamber 2 is configured for storage and retrieval, transfer, and tube picking of the samples in the sample storage chamber 1. A roller shutter passage assembly 3 is arranged on the sample storage chamber 1, and a sample scooping mechanism 4 is configured to scoop and transport the samples in the sample storage chamber 1 through the roller shutter passage assembly 3.

Specifically, the sample storage chamber 1 and the operation chamber 2 are provided; the operation chamber 2 is arranged laterally adjacent to the sample storage chamber 1, and the operation chamber 2 is configured for storage and retrieval, transfer, and tube picking of the samples in the sample storage chamber 1; and
the roller shutter passage assembly 3 is arranged on the sample storage chamber 1, and the roller shutter passage assembly 3 is arranged inside the operation chamber 2; and the sample scooping mechanism 4 is arranged inside the operation chamber 2, the roller shutter passage assembly 3 and the sample scooping mechanism 4 may move upward or downward, and the sample scooping mechanism 4 is configured to store and scoop the corresponding samples in the sample storage chamber 1 through the roller shutter passage assembly 3.

Preferably, the roller shutter passage assembly 3 may move upward or downward together with the sample scooping mechanism 4, thereby ensuring that the sample scooping mechanism 4 smoothly stores or scoops the samples through the roller shutter passage assembly 3.

In summary, in the present invention, the liftable roller shutter passage assembly 3 is arranged on the sample storage chamber 1, and a target sample is scooped by the liftable sample scooping mechanism 4, while other samples that do not need to be scooped are not exposed, thereby ensuring that internal samples will not lose viability due to temperature variations.

Further, the roller shutter passage assembly 3 includes a roller shutter 31 and a transfer window 32 opened on the roller shutter 31; and the roller shutter 31 is configured to drive the transfer window 32 to move upward or downward, and the sample scooping mechanism 4 is configured for storage and retrieval of the samples in the sample storage chamber 1 in a way of penetrating the transfer window 32.

Further, the roller shutter passage assembly 3 further includes a rolling storage member 33, the rolling storage member 33 is arranged on the roller shutter 31, and the rolling storage member 33 is configured to drive the roller shutter 31 to move upward or downward and retract the roller shutter 31.

Preferably, the roller shutter 31 is configured to retract the rolling storage member 33 in a way of moving upward or downward, vertical movement of the roller shutter 31 drives the transfer window 32 to move upward or downward, the transfer window 32 is configured to correspondingly match sample storage slots on each layer of a basket in the sample storage chamber 1, and the sample scooping mechanism 4 penetrates the transfer window 32 to store and retrieve or scoop the samples from the sample storage slots on each layer of the basket.

Preferably, the rolling storage member 33 drives the roller shutter 31 and the sample scooping mechanism 4 to move upward or downward simultaneously.

Preferably, the transfer window 32 is arranged to correspondingly match the sample storage slots on the basket, and other sample storage slots on the basket may be enclosed to reduce unnecessary temperature variations; and frequent storage and retrieval to samples does not adversely affect the samples in the basket.

Further, the rolling storage member 33 includes an upper roller shaft 331 and a lower roller shaft 332; the upper roller shaft 331 is arranged at an upper end of the roller shutter 31, the lower roller shaft 332 is arranged at a lower end of the roller shutter 31, the upper roller shaft 331 is configured to drive the roller shutter 31 to move upward for storage, and the lower roller shaft 332 is configured to drive the roller shutter 31 to move downward for storage.

Further, a grasping mechanism 5 is further arranged inside the operation chamber 2, the grasping mechanism 5 rotatably moves upward or downward to grasp a sample, and the grasping mechanism 5 may be butt-jointed with the sample scooping mechanism 4.

Preferably, the sample scooping mechanism 4 may move upward or downward to a side edge of the grasping mechanism 5 to cooperate with the grasping mechanism 5.

Further, the grasping mechanism 5 includes a grasping lifting assembly 51, a grasping rotating assembly 52, and a gripper member 53; and the grasping rotating assembly 52 is arranged on the grasping lifting assembly 51, the gripper member 53 is arranged on the grasping rotating assembly 52, the grasping lifting assembly 51 is configured to drive the grasping rotating assembly 52 to move upward or downward, the grasping rotating assembly 52 is configured to drive the gripper member 53 to rotate, and the gripper member 53 is configured to grasp the sample.

Further, a transfer tank retention zone is arranged at a side end of the grasping mechanism 5, a transfer tank 991 may be placed inside the transfer tank retention zone, and the grasping mechanism 5 is configured to grasp the samples in the transfer tank 991.

Further, a tank passage is opened on the operation chamber 2, the tank passage is arranged on one side of the transfer tank retention zone, a tank operation door 992 is arranged inside the tank passage, and the transfer tank 991 enters the transfer tank retention zone through the tank passage.

Preferably, the transfer tank 991 may be placed inside the transfer tank retention zone by opening the tank operation door 992, and the grasping mechanism 5 is configured to grasp the sample or place the grasped sample inside the transfer tank 991.

Further, a code scanning mechanism 6 is further arranged laterally adjacent to the grasping mechanism 5, and the grasping mechanism 5 is configured to grasp the sample and rotate same onto the code scanning mechanism 6 for code scanning.

Preferably, the code scanning mechanism 6 and the transfer tank retention zone are circumferentially arranged around the grasping mechanism 5, and the sample scooping mechanism 4 may move upward or downward to one side of the grasping mechanism 5; and the grasping mechanism 5 is capable of realizing the free movement of the above three functional areas, without the need to arrange an additional grasping mechanism 5, thereby saving costs.

Further, the sample scooping mechanism 4 includes a sample pick-and-place lifting assembly 41, a pick-and-place gripper assembly 42, and a steering support member 43, where the steering support member 43 is arranged on the sample pick-and-place lifting assembly 41, the pick-and-place gripper assembly 42 is arranged on the steering support member 43, the steering support member 43 may slide vertically on the sample pick-and-place lifting assembly 41, the steering support member 43 turns around the sample pick-and-place lifting assembly 41, and the pick-and-place gripper assembly 42 is configured to grasp and store the sample at a target position.

Preferably, at least one steering support member 43 is arranged, the pick-and-place gripper assembly 42 is configured to telescopically grasp the sample, and the pick-and-place gripper assembly 42 is configured to telescopically scoop a sample/biological plate rack in the sample storage chamber 1 through the transfer window 32; and alternatively, the sample/biological plate rack is placed inside the sample storage chamber 1.

Preferably, the sample pick-and-place lifting assembly 41 is configured to drive the steering support member 43 and the pick-and-place gripper assembly 42 to move upward or downward, and the steering support member 43 is configured to drive the pick-and-place gripper assembly 42 to change direction.

Further, the pick-and-place gripper assembly 42 includes a primary telescopic member 421, a secondary limiting and clamping member 422, and a grasping member 423, where the secondary limiting and clamping member 422 is arranged on the primary telescopic member 421, the grasping member 423 is arranged on the secondary limiting and clamping member 422, the grasping member 423 is configured to scoop the sample, the primary telescopic member 421 is configured to drive the secondary limiting and clamping member 422 and the grasping member 423 to perform telescopic movement, the secondary limiting and clamping member 422 is configured to perform secondary telescopic movement on the primary telescopic member 421, and the grasping member 423 is configured to perform telescopic movement on the secondary limiting and clamping member 422.

Preferably, the primary telescopic member 421 includes a mounting base, a motor, a gear, and a movable rack plate, where the motor and the gear are arranged on the mounting base, the movable rack plate is slidable on the mounting base, the motor drives the gear to rotate, the gear drives the movable rack plate to move forward or backward, the movable rack plate is connected to the secondary limiting and clamping member 422, and when the movable rack plate moves, the secondary limiting and clamping member 422 is driven to move forward or backward.

Preferably, through movement of the primary telescopic member 421 and the secondary limiting and clamping member 422, the sample or the sample plate rack is effectively scooped by the grasping member 423.

Further, the secondary limiting and clamping member 422 includes a secondary driving member 4221, a support frame 4222, and a limiting bar 4223, where the secondary driving member 4221 is configured to drive the support frame 4222 to move on the primary telescopic member 421, and the limiting bar 4223 is arranged on an inner side of the support frame 4222, and the limiting bar 4223 is configured to press against the grasping member 423.

Preferably, the secondary driving member 4221 may be composed of a magnetic motor, a slide rail, and a sliding block; and alternatively, the secondary driving member 4221 is a driving mechanism in the prior art as long as the driving function is achieved.

Further, the grasping member 423 includes a pushing member 4231, a scoop plate gripper 4232, and a tension-adjusting member 4233, where the tension-adjusting member 4233 is arranged on an inner side of the scoop plate gripper 4232, the pushing member 4231 is connected to the tension-adjusting member 4233, the limiting bar 4223 is configured to press against and limit the tension-adjusting member 4233 on the scoop plate gripper 4232, and the pushing member 4231 is configured to push the tension-adjusting member 4233 and the scoop plate gripper 4232 to disengage from the limiting bar 4223 so as to avoid being pressed and limited.

Preferably, the tension-adjusting member 4233 may be a spring, and the limiting bar 4223 inside the support frame 4222 is configured to press against the scoop plate gripper 4232, such that two scoop plate grippers 4232 move toward a center therebetween, thereby pressing against the plate rack or sample through the scoop plate grippers 4232.

Preferably, the pushing member 4231 is a hollow shaft motor and is configured to push the scoop plate gripper 4232 to disengage from the limiting bar 4223 on the support frame 4222, such that the tension-adjusting member 4233 is disengaged from the limiting bar 4223 to avoid being pressed, and the tension-adjusting member 4233 drives the two scoop plate grippers 4232 to expand outward, thereby releasing a clamped limiting state and facilitating the placement of the plate rack or sample to a specific position.

Preferably, the pushing member 4231 is configured to push the scoop plate gripper 4232 into the support frame 4222, and the limiting bar 4223 is configured to press against the tension-adjusting member 4233, such that the sample is clamped; and alternatively, the secondary driving member 4221 is configured to drive the support frame 4222 to clamp the scoop plate gripper 4232, depending on conditions inside the chamber.

Further, a thermal insulation door 7 is arranged inside the operation chamber 2, and the thermal insulation door 7 is configured to open and close the roller shutter passage assembly 3.

Preferably, when sample storage and retrieval is not required, the roller shutter passage assembly 3 is closed by the thermal insulation door 7, to further insulate the sample storage chamber 1.

Further, a basket 8 is arranged inside the sample storage chamber 1, and the transfer window 32 moves upward or downward to correspondingly match the storage slots on the basket 8.

Preferably, the 360-degree rotatable basket 8 may be arranged inside the sample storage chamber 1.

Preferably, a plurality of sample storage slots are vertically arranged on the basket 8, and the sample or sample plate rack is stored in each of the storage slots.

Further, an observation window 993 and an operation terminal 994 are arranged on the operation chamber 2, where the observation window 993 is configured to observe an internal operating state of the operation chamber 2, and the operation terminal 994 is configured to issue commands and display an internal state of the sample storage chamber 1.

In summary, in the present invention, the rolling storage member 33 drives the transfer window 32 to move upward or downward, so as to match the corresponding storage slot on the basket in the sample storage chamber 1, and the sample scooping mechanism 4 penetrates the transfer window 32 by moving upward or downward to scoop the sample on the basket, which avoids exposure of other samples that do not need to be extracted, and reduces the adverse effects on the samples in the sample storage chamber 1; the grasping mechanism 5, the transfer tank retention zone and the code scanning mechanism 6 are arranged together, and the sample scooping mechanism 4 may move upward or downward to a side of the grasping mechanism 5, such that the grasping mechanism 5 facilitates sample grasping, transport and scanning, and only one grasping mechanism 5 is needed to realize the free movement of a plurality of functional areas; the thermal insulation door 7 is configured to close the roller shutter passage assembly 3, so as to further insulate the sample storage chamber 1; and the sample scooping mechanism 4 is configured to achieve sample storage and retrieval, and it is ensured that the sample does not fall off during the scooping and transport process, and after being transported to a specific position, the sample or plate rack is pushed out and placed.

Importantly, it should be noted that the construction and arrangement of the present invention shown in a plurality of different exemplary embodiments are only illustrative. Although only a few embodiments are described in detail in the disclosed content, those who refer to the disclosed content should readily understood that many modifications can be made (e.g., variations in dimensions, scales, structures, shapes and proportions of various elements, parameters (e.g., temperature, pressure, and the like), installation arrangement, material selections, colors, orientations, and the like) without departing from the scope of the invention as defined in the appended claims.

For example, the element shown as integrally formed may be composed of a plurality of parts or elements, the position of the element may be inverted or otherwise changed, and the nature, number, or position of the discrete element may be modified or varied.

Additionally, in order to provide a concise description of exemplary embodiments, not all features of an actual embodiment (i.e., those features unrelated to the best mode presently contemplated for carrying out the present invention or those features unrelated to implementing of the present invention) must be described.

It should be understood that in the development of any actual embodiment, numerous implementation-specific decisions may be made in any engineering or design project. Such development efforts may be complex and time-consuming, but for those skilled in the art benefiting from the disclosed content, such efforts will be routine design, manufacturing, and production without excessive experimentation.

It should be noted that the above embodiments are merely intended for describing the technical solutions of the present invention rather than limiting same. Although the present invention is described in detail with reference to the above embodiments, persons of ordinary skill in the art should understand that they may still make modifications, which shall fall within the scope of the claims of the present invention.

## Claims

1. A vertical low-temperature storage refrigerator, comprising a sample storage chamber (1) and an operation chamber (2), wherein the operation chamber (2) is arranged laterally adjacent to the sample storage chamber (1), and the operation chamber (2) is configured for storage and retrieval, and transfer of samples in the sample storage chamber (1), wherein a grasping mechanism (5) is arranged inside the operation chamber (2), the grasping mechanism (5) being configured to rotatably move upward or downward to grasp a sample, and the grasping mechanism (5) being configured to be butt-jointed with a
sample scooping mechanism (4);
a roller shutter passage assembly (3) is arranged on the sample storage chamber (1), and the roller shutter passage assembly (3) is arranged inside the operation chamber (2); and the sample scooping mechanism (4) is arranged inside the operation chamber (2), the roller shutter passage assembly (3) and the sample scooping mechanism (4) are allowed to move upward or downward, and the sample scooping mechanism (4) is configured to store and scoop corresponding samples in the sample storage chamber (1) through the roller shutter passage assembly (3); and
the roller shutter passage assembly (3) comprises a roller shutter (31) and a transfer window (32) opened on the roller shutter (31); and the roller shutter (31) is configured to drive the transfer window (32) to move upward or downward, and the sample scooping mechanism (4) is configured for storage and retrieval of the samples in the sample storage chamber (1) in a way of penetrating the transfer window (32).

2. The vertical low-temperature storage refrigerator according to claim 1, **characterized in that** the roller shutter passage assembly (3) further comprises a rolling storage member (33), the rolling storage member (33) is arranged on the roller shutter (31), and the rolling storage member (33) is configured to drive the roller shutter (31) to move upward or downward and retract the roller shutter (31).

3. The vertical low-temperature storage refrigerator according to claim 2, **characterized in that** the rolling storage member (33) comprises an upper roller shaft (331) and a lower roller shaft (332), the upper roller shaft (331) is arranged at an upper end of the roller shutter (31), the lower roller shaft (332) is arranged at a lower end of the roller shutter (31), the upper roller shaft (331) is configured to drive the roller shutter (31) to move upward for storage, and the lower roller shaft (332) is configured to drive the roller shutter (31) to move downward for storage.

4. The vertical low-temperature storage refrigerator according to claim 1, **characterized in that** the grasping mechanism (5) comprises a grasping lifting assembly (51), a grasping rotating assembly (52), and a gripper member (53); and the grasping rotating assembly (52) is arranged on the grasping lifting assembly (51), the gripper member (53) is arranged on the grasping rotating assembly (52), the grasping lifting assembly (51) is configured to drive the grasping rotating assembly (52) to move upward or downward, the grasping rotating assembly (52) is configured to drive the gripper member (53) to rotate, and the gripper member (53) is configured to grasp the sample.

5. The vertical low-temperature storage refrigerator according to claim 1, **characterized in that** a transfer tank retention zone is arranged at a side end of the grasping mechanism (5), a transfer tank (991) is placed inside the transfer tank retention zone, and the grasping mechanism (5) is configured to grasp the samples in the transfer tank (991).

6. The vertical low-temperature storage refrigerator according to claim 5, **characterized in that** a tank passage is opened on the operation chamber (2), the tank passage is arranged on one side of the transfer tank retention zone, a tank operation door (992) is arranged inside the tank passage, and the transfer tank (991) enters the transfer tank retention zone through the tank passage.

7. The vertical low-temperature storage refrigerator according to claim 1, **characterized in that** a code scanning mechanism (6) is further arranged laterally adjacent to the grasping mechanism (5), and the grasping mechanism (5) is configured to grasp the sample and rotate same onto the code scanning mechanism (6) for code scanning.

8. The vertical low-temperature storage refrigerator according to claim 1, **characterized in that** the sample scooping mechanism (4) comprises a sample pick-and-place lifting assembly (41), a pick-and-place gripper assembly (42), and a steering support member (43), wherein the steering support member (43) is arranged on the sample pick-and-place lifting assembly (41), the pick-and-place gripper assembly (42) is arranged on the steering support member (43), the steering support member (43) slides vertically on the sample pick-and-place lifting assembly (41), the steering support member (43) turns around the sample pick-and-place lifting assembly (41), and the pick-and-place gripper assembly (42) is configured to grasp and store the sample at a target position.

9. The vertical low-temperature storage refrigerator according to claim 8, **characterized in that** the pick-and-place gripper assembly (42) comprises a primary telescopic member (421), a secondary limiting and clamping member (422), and a grasping member (423), wherein the secondary limiting and clamping member (422) is arranged on the primary telescopic member (421), the grasping member (423) is arranged on the secondary limiting and clamping member (422), the grasping member (423) is configured to scoop the sample, the primary telescopic member (421) is configured to drive the secondary limiting and clamping member (422) and the grasping member (423) to perform telescopic movement, the secondary limiting and clamping member (422) is configured to perform secondary telescopic movement on the primary telescopic member (421), and the grasping member (423) is configured to perform telescopic movement on the secondary limiting and clamping member (422).

10. The vertical low-temperature storage refrigerator according to claim 9, **characterized in that** the secondary limiting and clamping member (422) comprises a secondary driving member (4221), a support frame (4222), and a limiting bar (4223), wherein the secondary driving member (4221) is configured to drive the support frame (4222) to move on the primary telescopic member (421), and the limiting bar (4223) is arranged on an inner side of the support frame (4222), and the limiting bar (4223) is configured to press against the grasping member (423).

11. The vertical low-temperature storage refrigerator according to claim 10, **characterized in that** the grasping member (423) comprises a pushing member (4231), a scoop plate gripper (4232), and a tension-adjusting member (4233), wherein the tension-adjusting member (4233) is arranged on an inner side of the scoop plate gripper (4232), the pushing member (4231) is connected to the tension-adjusting member (4233), the limiting bar (4223) is configured to press against and limit the tension-adjusting member (4233) on the scoop plate gripper (4232), and the pushing member (4231) is configured to push the tension-adjusting member (4233) and the scoop plate gripper (4232) to disengage from the limiting bar (4223) so as to avoid being pressed and limited.

12. The vertical low-temperature storage refrigerator according to claim 1, **characterized in that** a thermal insulation door (7) is arranged inside the operation chamber (2), and the thermal insulation door (7) is configured to open and close the roller shutter passage assembly (3).

13. The vertical low-temperature storage refrigerator according to claim 1, **characterized in that** a basket (8) is arranged inside the sample storage chamber (1), and the transfer window (32) moves upward or downward to correspondingly match a storage slot on the basket (8).

14. The vertical low-temperature storage refrigerator according to claim 1, **characterized in that** an observation window (993) and an operation terminal (994) are arranged on the operation chamber (2), the observation window (993) allows to observe an internal operating state of the operation chamber (2), and the operation terminal (994) is configured to issue commands and display an internal state of the sample storage chamber (1).

## Patentansprüche

1. Vertikaler Tieftemperatur-Lagerkühlschrank, umfassend eine Probenlagerkammer (1) und eine Betriebskammer (2), wobei die Betriebskammer (2) seitlich benachbart zu der Probenlagerkammer (1) angeordnet ist, und wobei die Betriebskammer (2) zum Lagern, Entnehmen und Transferieren von Proben in der Probenlagerkammer (1) ausgebildet ist, wobei in der Betriebskammer (2) ein Greifmechanismus (5) angeordnet ist, wobei der Greifmechanismus (5) so ausgebildet ist, sich drehbar aufwärts oder abwärts zu bewegen, um eine Probe zu greifen, und wobei der Greifmechanismus (5) so ausgebildet ist, mit einem Probenaufnahmemechanismus (4) stumpf verbunden zu werden;
wobei an der Probenlagerkammer (1) eine Rollladen-Durchgangsanordnung (3) angeordnet ist, wobei die Rollladen-Durchgangsanordnung (3) in der Betriebskammer (2) angeordnet ist; und wobei der Probenaufnahmemechanismus (4) in der Betriebskammer (2) angeordnet ist, wobei sich die Rollladen-Durchgangsanordnung (3) und der Probenaufnahmemechanismus (4) aufwärts oder abwärts bewegen können, und wobei der Probenaufnahmemechanismus (4) so ausgebildet ist, entsprechende Proben in der Probenlagerkammer (1) durch die Rollladen-Durchgangsanordnung (3) zu lagern und aufzunehmen; und
wobei die Rollladen-Durchgangsanordnung (3) einen Rollladen (31) und ein an dem Rollladen (31) ausgebildetes Transferfenster (32) umfasst; und wobei der Rollladen (31) so ausgebildet ist, das Transferfenster (32) zu einer Aufwärts- oder Abwärtsbewegung zu veranlassen, und wobei der Probenaufnahmemechanismus (4) zum Lagern und Entnehmen der Proben in der Probenlagerkammer (1) durch Hindurchdringen durch das Transferfenster (32) ausgebildet ist.

2. Vertikaler Tieftemperatur-Lagerkühlschrank nach Anspruch 1, **dadurch gekennzeichnet, dass** die Rollladen-Durchgangsanordnung (3) ferner ein Aufrolllagerelement (33) umfasst, wobei das Aufrolllagerelement (33) an dem Rollladen (31) angeordnet ist, und wobei das Aufrolllagerelement (33) so ausgebildet ist, den Rollladen (31) zu einer Aufwärts- oder Abwärtsbewegung zu veranlassen und den Rollladen (31) einzuziehen.

3. Vertikaler Tieftemperatur-Lagerkühlschrank nach Anspruch 2, **dadurch gekennzeichnet, dass** das Aufrolllagerelement (33) eine obere Rollenwelle (331) und eine untere Rollenwelle (332) umfasst, wobei die obere Rollenwelle (331) an einem oberen Ende des Rollladens (31) angeordnet ist, wobei die untere Rollenwelle (332) an einem unteren Ende des Rollladens (31) angeordnet ist, wobei die obere Rollenwelle (331) so ausgebildet ist, den Rollladen (31) zum Zwecke des Speicherns aufwärts zu bewegen, und wobei die untere Rollenwelle (332) so ausgebildet ist, den Rollladen (31) zum Zwecke des Speicherns abwärts zu bewegen.

4. Vertikaler Tieftemperatur-Lagerkühlschrank nach Anspruch 1, **dadurch gekennzeichnet, dass** der Greifmechanismus (5) eine Greifhubanordnung (51), eine Greifdrehanordnung (52) und ein Greiferelement (53) umfasst; und wobei die Greifdrehanordnung (52) an der Greifhubanordnung (51) angeordnet ist, wobei das Greiferelement (53) an der Greifdrehanordnung (52) angeordnet ist, wobei die Greifhubanordnung (51) so ausgebildet ist, die Greifdrehanordnung (52) zu einer Aufwärts- oder Abwärtsbewegung zu veranlassen, wobei die Greifdrehanordnung (52) so ausgebildet ist, das Greiferelement (53) zu drehen, und wobei das Greiferelement (53) so ausgebildet ist, die Probe zu greifen.

5. Vertikaler Tieftemperatur-Lagerkühlschrank nach Anspruch 1, **dadurch gekennzeichnet, dass** an einem seitlichen Ende des Greifmechanismus (5) eine Transferbehälter-Aufnahmezone angeordnet ist, wobei in der Transferbehälter-Aufnahmezone ein Transferbehälter (991) platziert ist, und wobei der Greifmechanismus (5) so ausgebildet ist, die Proben in dem Transferbehälter (991) zu greifen.

6. Vertikaler Tieftemperatur-Lagerkühlschrank nach Anspruch 5, **dadurch gekennzeichnet, dass** an der Betriebskammer (2) ein Behälterdurchlass ausgebildet ist, wobei der Behälterdurchlass an einer Seite der Transferbehälter-Aufnahmezone angeordnet ist, wobei in dem Behälterdurchlass eine Behälterbedientür (992) angeordnet ist, und wobei der Transferbehälter (991) durch den Behälterdurchlass in die Transferbehälter-Aufnahmezone gelangt.

7. Vertikaler Tieftemperatur-Lagerkühlschrank nach Anspruch 1, **dadurch gekennzeichnet, dass** seitlich benachbart zu dem Greifmechanismus (5) ferner ein Codescanmechanismus (6) angeordnet ist, und wobei der Greifmechanismus (5) so ausgebildet ist, die Probe zu greifen und diese auf den Codescanmechanismus (6) zu drehen, um ein Codescannen durchzuführen.

8. Vertikaler Tieftemperatur-Lagerkühlschrank nach Anspruch 1, **dadurch gekennzeichnet, dass** der Probenaufnahmemechanismus (4) eine Probenbe- und entladehubanordnung (41), eine Be- und Entladegreiferanordnung (42) und ein Schwenkstützelement (43) umfasst, wobei das Schwenkstützelement (43) an der Probenbe- und entladehubanordnung (41) angeordnet ist, wobei die Be- und Entladegreiferanordnung (42) an dem Schwenkstützelement (43) angeordnet ist, wobei das Schwenkstützelement (43) an der Probenbe- und entladehubanordnung (41) vertikal gleitet, wobei sich das Schwenkstützelement (43) um die Probenbe- und entladehubanordnung (41) dreht, und wobei die Be- und Entladegreiferanordnung (42) so ausgebildet ist, die Probe an einer Zielposition zu greifen und zu lagern.

9. Vertikaler Tieftemperatur-Lagerkühlschrank nach Anspruch 8, **dadurch gekennzeichnet, dass** die Be- und Entladegreiferanordnung (42) ein primäres Teleskopelement (421), ein sekundäres Begrenzungs- und Klemmelement (422) und ein Greifelement (423) umfasst, wobei das sekundäre Begrenzungs- und Klemmelement (422) an dem primären Teleskopelement (421) angeordnet ist, wobei das Greifelement (423) an dem sekundären Begrenzungs- und Klemmelement (422) angeordnet ist, wobei das Greifelement (423) so ausgebildet ist, die Probe aufzunehmen, wobei das primäre Teleskopelement (421) so ausgebildet ist, das sekundäre Begrenzungs- und Klemmelement (422) und das Greifelement (423) zu einer Teleskopbewegung zu veranlassen, wobei das sekundäre Begrenzungs- und Klemmelement (422) so ausgebildet ist, eine sekundäre Teleskopbewegung auf dem primären Teleskopelement (421) durchzuführen, und wobei das Greifelement (423) so ausgebildet ist, eine Teleskopbewegung an dem sekundären Begrenzungs- und Klemmelement (422) durchzuführen.

10. Vertikaler Tieftemperatur-Lagerkühlschrank nach Anspruch 9, **dadurch gekennzeichnet, dass** das sekundäre Begrenzungs- und Klemmelement (422) ein sekundäres Antriebselement (4221), ein Traggestell (4222) und eine Begrenzungsstange (4223) umfasst, wobei das sekundäre Antriebselement (4221) so ausgebildet ist, das Traggestell (4222) zu einer Bewegung auf dem primären Teleskopelement (421) zu veranlassen, und wobei die Begrenzungsstange (4223) an einer Innenseite des Traggestells (4222) angeordnet ist, und wobei die Begrenzungsstange (4223) so ausgebildet ist, gegen das Greifelement (423) zu drücken.

11. Vertikaler Tieftemperatur-Lagerkühlschrank nach Anspruch 10, **dadurch gekennzeichnet, dass** das Greifelement (423) ein Schubelement (4231), einen Schöpfplattengreifer (4232) und ein Spannelement (4233) umfasst, wobei das Spannelement (4233) an einer Innenseite des Schöpfplattengreifers (4232) angeordnet ist, wobei das Schubelement (4231) mit dem Spannelement (4233) verbunden ist, wobei die Begrenzungsstange (4223) so ausgebildet ist, das Spannelement (4233) an dem Schöpfplattengreifer (4232) zu drücken und zu begrenzen, und wobei das Schubelement (4231) so ausgebildet ist, das Spannelement (4233) und den Schöpfplattengreifer (4232) dazu zu veranlassen, sich von der Begrenzungsstange (4223) zu lösen, um zu vermeiden, dass diese gedrückt und begrenzt werden.

12. Vertikaler Tieftemperatur-Lagerkühlschrank nach Anspruch 1, **dadurch gekennzeichnet, dass** in der Betriebskammer (2) eine Wärmedämmtür (7) angeordnet ist, und wobei die Wärmedämmtür (7) so ausgebildet ist, die Rollladen-Durchgangsanordnung (3) zu öffnen und zu schließen.

13. Vertikaler Tieftemperatur-Lagerkühlschrank nach Anspruch 1, **dadurch gekennzeichnet, dass** in der Probenlagerkammer (1) ein Korb (8) angeordnet ist, und wobei sich das Transferfenster (32) aufwärts oder abwärts bewegt, um entsprechend mit einem Lagerfach an dem Korb (8) übereinzustimmen.

14. Vertikaler Tieftemperatur-Lagerkühlschrank nach Anspruch 1, **dadurch gekennzeichnet, dass** an der Betriebskammer (2) ein Beobachtungsfenster (993) und ein Bedienterminal (994) angeordnet sind, wobei das Beobachtungsfenster (993) ein Beobachten eines inneren Betriebszustands der Betriebskammer (2) ermöglicht, und wobei das Bedienterminal (994) so ausgebildet ist, Befehle auszugeben und einen inneren Zustand der Probenlagerkammer (1) anzuzeigen.

## Revendications

1. Un réfrigérateur vertical de stockage à basse température, comprenant une chambre de stockage d'échantillons (1) et une chambre de fonctionnement (2), dans lequel la chambre de fonctionnement (2) est disposée latéralement de manière adjacente à la chambre de stockage d'échantillons (1), et la chambre de fonctionnement (2) est configurée pour assurer le stockage, la récupération et le transfert d'échantillons dans la chambre de stockage d'échantillons (1), un mécanisme de préhension (5) étant disposé à l'intérieur de la chambre de fonctionnement (2), le mécanisme de préhension (5) étant configuré pour se déplacer en rotation vers le haut ou vers le bas afin de saisir un échantillon, et le mécanisme de préhension (5) étant configuré pour s'accoupler bout à bout avec un mécanisme de prélèvement d'échantillons (4) ;
un ensemble de passage à volet roulant (3) est disposé sur la chambre de stockage d'échantillons (1), et l'ensemble de passage à volet roulant (3) est disposé à l'intérieur de la chambre de fonctionnement (2) ; et le mécanisme de prélèvement d'échantillons (4) est disposé à l'intérieur de la chambre de fonctionnement (2), l'ensemble de passage à volet roulant (3) et le mécanisme de prélèvement d'échantillons (4) pouvant se déplacer vers le haut ou vers le bas, et le mécanisme de prélèvement d'échantillons (4) étant configuré pour stocker et prélever les échantillons correspondants dans la chambre de stockage d'échantillons (1) par l'intermédiaire de l'ensemble de passage à volet roulant (3) ; et
l'ensemble de passage à volet roulant (3) comprend un volet roulant (31) et une fenêtre de transfert (32) ménagée dans le volet roulant (31) ; et le volet roulant (31) est configuré pour entraîner la fenêtre de transfert (32) vers le haut ou vers le bas, et le mécanisme de prélèvement d'échantillons (4) est configuré pour assurer le stockage et la récupération des échantillons dans la chambre de stockage d'échantillons (1) en traversant la fenêtre de transfert (32).

2. Le réfrigérateur vertical de stockage à basse température selon la revendication 1, **caractérisé en ce que** l'ensemble de passage à volet roulant (3) comprend en outre un organe d'enroulement (33), l'organe d'enroulement (33) étant disposé sur le volet roulant (31), et l'organe d'enroulement (33) étant configuré pour entraîner le volet roulant (31) vers le haut ou vers le bas et rétracter le volet roulant (31).

3. Le réfrigérateur vertical de stockage à basse température selon la revendication 2, **caractérisé en ce que** l'organe d'enroulement (33) comprend un axe d'enroulement supérieur (331) et un axe d'enroulement inférieur (332), l'axe d'enroulement supérieur (331) étant disposé à une extrémité supérieure du volet roulant (31), l'axe d'enroulement inférieur (332) étant disposé à une extrémité inférieure du volet roulant (31), l'axe d'enroulement supérieur (331) étant configuré pour entraîner le volet roulant (31) vers le haut afin de le ranger, et l'axe d'enroulement inférieur (332) étant configuré pour entraîner le volet roulant (31) vers le bas afin de le ranger.

4. Le réfrigérateur vertical de stockage à basse température selon la revendication 1, **caractérisé en ce que** le mécanisme de préhension (5) comprend un ensemble de levage de préhension (51), un ensemble de rotation de préhension (52) et un organe de préhension (53) ; l'ensemble de rotation de préhension (52) étant disposé sur l'ensemble de levage de préhension (51), l'organe de préhension (53) étant disposé sur l'ensemble de rotation de préhension (52), l'ensemble de levage de préhension (51) étant configuré pour entraîner l'ensemble de rotation de préhension (52) vers le haut ou vers le bas, l'ensemble de rotation de préhension (52) étant configuré pour entraîner l'organe de préhension (53) en rotation, et l'organe de préhension (53) étant configuré pour saisir l'échantillon.

5. Le réfrigérateur vertical de stockage à basse température selon la revendication 1, **caractérisé en ce qu'**une zone de maintien de la cuve de transfert est disposée à une extrémité latérale du mécanisme de préhension (5), une cuve de transfert (991) est placée à l'intérieur de la zone de maintien de la cuve de transfert, et le mécanisme de préhension (5) est configuré pour saisir les échantillons dans la cuve de transfert (991).

6. Le réfrigérateur vertical de stockage à basse température selon la revendication 5, **caractérisé en ce qu'**un passage pour la cuve est ménagé dans la chambre de fonctionnement (2), le passage pour la cuve étant disposé d'un côté de la zone de maintien de la cuve de transfert, une porte d'accès (992) étant disposée à l'intérieur du passage pour la cuve, et la cuve de transfert (991) pénétrant dans la zone de maintien de la cuve de transfert par le passage pour la cuve.

7. Le réfrigérateur vertical de stockage à basse température selon la revendication 1, **caractérisé en ce qu'**un mécanisme de lecture de code (6) est en outre disposé latéralement à côté du mécanisme de préhension (5), et le mécanisme de préhension (5) est configuré pour saisir l'échantillon et le faire pivoter vers le mécanisme de lecture de code (6) afin d'en lire le code.

8. Le réfrigérateur vertical de stockage à basse température selon la revendication 1, **caractérisé en ce que** le mécanisme de prélèvement d'échantillons (4) comprend un ensemble de levage de prise et de dépose d'échantillons (41), un ensemble de préhension de prise et de dépose (42) et un organe de support d'orientation (43), dans lequel l'organe de support d'orientation (43) est disposé sur l'ensemble de levage de prise et de dépose d'échantillons (41), l'ensemble de préhension de prise et de dépose (42) est disposé sur l'organe de support d'orientation (43), l'organe de support d'orientation (43) coulisse verticalement sur l'ensemble de levage de prise et de dépose d'échantillons (41), l'organe de support d'orientation (43) tourne autour de l'ensemble de levage de prise et de dépose d'échantillons (41), et l'ensemble de préhension de prise et de dépose (42) est configuré pour saisir et stocker l'échantillon à une position cible.

9. Le réfrigérateur vertical de stockage à basse température selon la revendication 8, **caractérisé en ce que** l'ensemble de préhension de prise et de dépose (42) comprend un organe télescopique primaire (421), un organe secondaire de limitation et de serrage (422) et un organe de préhension (423), dans lequel l'organe secondaire de limitation et de serrage (422) est disposé sur l'organe télescopique primaire (421), l'organe de préhension (423) est disposé sur l'organe secondaire de limitation et de serrage (422), l'organe de préhension (423) est configuré pour prélever l'échantillon, l'organe télescopique primaire (421) est configuré pour entraîner l'organe secondaire de limitation et de serrage (422) et l'organe de préhension (423) dans un mouvement télescopique, l'organe secondaire de limitation et de serrage (422) est configuré pour effectuer un mouvement télescopique secondaire sur l'organe télescopique primaire (421), et l'organe de préhension (423) est configuré pour effectuer un mouvement télescopique sur l'organe secondaire de limitation et de serrage (422).

10. Le réfrigérateur vertical de stockage à basse température selon la revendication 9, **caractérisé en ce que** l'organe secondaire de limitation et de serrage (422) comprend un organe d'entraînement secondaire (4221), un châssis de support (4222) et une barre de limitation (4223), dans lequel l'organe d'entraînement secondaire (4221) est configuré pour entraîner le châssis de support (4222) en déplacement sur l'organe télescopique primaire (421), et la barre de limitation (4223) est disposée sur un côté intérieur du châssis de support (4222), et la barre de limitation (4223) est configurée pour venir en appui contre l'organe de préhension (423).

11. Le réfrigérateur vertical de stockage à basse température selon la revendication 10, **caractérisé en ce que** l'organe de préhension (423) comprend un organe de poussée (4231), une pince à plaque de prélèvement (4232) et un organe de réglage de la tension (4233), dans lequel l'organe de réglage de la tension (4233) est disposé sur un côté intérieur de la pince à plaque de prélèvement (4232), l'organe de poussée (4231) est relié à l'organe de réglage de la tension (4233), la barre de limitation (4223) est configurée pour venir en appui contre l'organe de réglage de la tension (4233) situé sur la pince à plaque de prélèvement (4232) et le limiter, et l'organe de poussée (4231) est configuré pour pousser l'organe de réglage de la tension (4233) et la pince à plaque de prélèvement (4232) afin de les désengager de la barre de limitation (4223), de sorte qu'ils ne soient plus soumis à son appui ni à sa limitation.

12. Le réfrigérateur vertical de stockage à basse température selon la revendication 1, **caractérisé en ce qu'**une porte d'isolation thermique (7) est disposée à l'intérieur de la chambre de fonctionnement (2), et la porte d'isolation thermique (7) est configurée pour ouvrir et fermer l'ensemble de passage à volet roulant (3).

13. Le réfrigérateur vertical de stockage à basse température selon la revendication 1, **caractérisé en ce qu'**un panier (8) est disposé à l'intérieur de la chambre de stockage d'échantillons (1), et la fenêtre de transfert (32) se déplace vers le haut ou vers le bas de manière à s'aligner avec un emplacement de stockage correspondant du panier (8).

14. Le réfrigérateur vertical de stockage à basse température selon la revendication 1, **caractérisé en ce qu'**une fenêtre d'observation (993) et un terminal de commande (994) sont disposés sur la chambre de fonctionnement (2), la fenêtre d'observation (993) permettant d'observer l'état de fonctionnement interne de la chambre de fonctionnement (2), et le terminal de commande (994) étant configuré pour émettre des commandes et afficher l'état interne de la chambre de stockage d'échantillons (1).
